# EUROPEAN PATENT APPLICATION

(11) **EP 3 928 637 A1**
(43) Date of publication of application: **29.12.2021**
(21) Application number: 20760061.0
(22) Date of filing: 20.02.2020
(51) Int. Cl.: A23L 33/135, A61K 35/747, A61P 25/24, A61P 25/22

(54) **KIMCHI LACTOBACILLUS SAKEI HAVING PROPHYLACTIC, AMELIORATING OR THERAPEUTIC EFFECT ON DEPRESSION AND ANXIETY DISORDERS**

(30) Priority: 22.02.2019 KR 20190021068
(71) Applicant: Korea Food Research Institute, Wanju-gun, Jeollabuk-do 55365 (KR)
(72) Inventor: CHOI, Hak Jong, Gwangju 62065 (KR); KIM, Nam Hee, Gwangju 62059 (KR); PARK, Hyo Kyeong, Gwangju 61005 (KR); LEE, Ji Eun, Busan 47504 (KR)
(74) Representative: V.O.
(86) International application number: PCT/KR2020/002490
(87) International publication number: WO 2020/171633

(57) **Abstract**

The present disclosure relates to a composition for preventing, alleviating or treating depression or anxiety disorder, which contains *Lactobacillus sakei* as an active ingredient. The *Lactobacillus sakei* strain according to the present disclosure can be used to prevent, alleviate or treat a mental disorder such as depression or anxiety disorder.

## Description

### [Technical Field]

The present disclosure relates to *Lactobacillus sakei* having prophylactic, alleviating or therapeutic effect on depression and anxiety disorder.

### [Background Art]

Modern people are exposed to a lot of stress due to intricate social structure. As a result, the number of people who suffer from mental disorders caused by various stresses, such as depression, anxiety disorder, sleep disorder, etc., is increasing rapidly.

If the stress is left untreated, it results in psychological responses such as depression, anxiety, fatigue, anger and mood change as well as physiological responses such as change in blood pressure, increased pulse rate, etc. If such responses are repeated consistently, they lead to diseases such as depression, anxiety and sleep disorder, placing burdens on individuals, families and societies and lowering the quality of individual lives.

Among the diseases caused by stress, depression refers to a state of low mood characterized by sorrow, despair and discouragement. That is to say, it refers to the state that has developed from the normal emotion of "gloominess" through dysthymic disorder to major depressive disorder. The main symptom is major depressive episode which refers to the depressed mood or loss of interest or pleasure in most activities in association with change in appetite, body weight or sleeping pattern, psychomotor agitation or retardation, decreased thinking ability, attention or decision-making ability, decreased activity and fatigue, feeling of worthlessness, remorse or guilt, frequent thought about death or suicide, or suicidal plan or attempt.

All the antidepressants currently used clinically are drugs that enhance the action of monoamine neurotransmitters, including substances that inhibit serotonin reuptake, norepinephrine reuptake, etc. of nerve cells (SSRIs, SNRIs, TCAs, etc.) or substances that inhibit the degradation of serotonin, dopamine, etc. such as monoamine oxidase inhibitors.

However, only about 10-25% of patients with depression are taking antidepressants in Korea at present, and it is known that approximately 40% of them stop medication owing to decreased compliance due to side effects, etc. In addition, 33% of those who take antidepressants are reported to be non-responders. Therefore, a composition with antidepressant effect derived from a natural product is needed desperately.

In addition, depression may also be accompanied by other diseases. For example, depression is often accompanied by neurodegenerative diseases. The neurodegenerative diseases refer to degenerative diseases occurring with aging, particularly in the brain. Neurodegenerative diseases may be classified depending on main symptoms and the affected part of the brain. Typical examples are Alzheimer's disease and Parkinson's disease. Although it is known that neurodegenerative diseases are caused by neurodegeneration due to aging and aggregation of proteins owing to genetic and environmental factors, which lead to the death of nerve cells, the exact cause is not known yet.

Parkinson's disease is the second most prevalent neurodegenerative disease after Alzheimer's disease. It is reported that about 1% of the people aged 50 years or older suffer from this disease. The main symptoms of Parkinson's disease are tremor, rigidity, slowness of movement, postural instability, etc. It is a chronic disease caused by the deficit of a neurotransmitter called dopamine in the brain. Dopamine is produced by nerve cells in the substantia nigra of the brain. The nerve cells of the substantia nigra are intricately connected to the motor cortex and other various parts of the brain. Parkinson's disease is caused by the deficit of dopamine which is a substance secreted for regulation of the function of the basal ganglia in the substantia nigra.

The representative symptoms of Parkinson's disease are motor symptoms such as 1) slowness of movement (bradykinesia), 2) tremor at rest, 3) muscle rigidity, 4) loss of postural reflexes, 5) abnormally flexed posture, 6) freezing of gait, etc. Other symptoms that may occur during the progression of Parkinson's disease may occur not only autonomic disturbance, sleep disorder and sensory disturbance of smell or temperature but also depression and cognitive disorder. In particular, mental symptoms such as depression and anxiety disorder occur in 40-50% of patients suffering from Parkinson's disease and are pointed out as main factors that aggravate the quality of life.

The currently available therapies for Parkinson's disease include drug therapy, surgery, physical therapy, etc. For drug therapy, drugs which boost the depleted level of dopamine in the brain, prevent or delay the destruction of nerve cells by neutralizing the imbalance in neurotransmitters caused by the deficit of dopamine and control other symptoms such as depression, etc. are generally used. For example, dopamine-replacing drugs such as L-DOPA or drugs that act on the dopamine receptor are used for the treatment of Parkinson's disease. However, these drugs are limited in that they aim only at the control of symptoms since it is difficult to regenerate the dead nerve cells and that long-term medication results in severe side effects such as involuntary movement (dyskinesia), vomiting, etc. Accordingly, development of a drug that that alleviates symptoms and provides neuroprotective effect with ensured safety is imminent.

In particular, it was recently reported that the change in gut microbiota induced in patients with Parkinson's disease is pathophysiologically associated with the progress of Parkinson's disease. Moreover, it was found out that fecal microbiota transplantation (FMT) to an animal model of Parkinson's disease resulted in neuroprotective effect through regulation of neuroinflammation (non-patent document 1).

Under this background, prevention and treatment of Parkinson's disease using lactic acid bacteria are studied recently. However, lactic acid bacteria that directly affect the symptoms of ataxia, depression or anxiety disorder of Parkinson's disease, except constipation, have not been found yet. Therefore, development of lactic acid bacteria capable of alleviating or treating the symptoms of ataxia, depression or anxiety disorder caused by Parkinson's disease is necessary.

### [References of Related Art]

### [Non-patent Documents]

(Non-patent document 1) Xin Fang, Microbial treatment: the potential application for Parkinson's disease, Neurological Sciences, 2018.

### [Disclosure]

### [Technical Problem]

The present disclosure is directed to providing a *Lactobacillus sakei* strain having prophylactic, alleviating or therapeutic effect on depression or anxiety disorder.

### [Technical Solution]

The inventors of the present disclosure have made efforts to find a strain which has an effect of improving the symptoms of depression or anxiety disorder. As a result, they have identified that the administration of a *Lactobacillus sakei* strain isolated from kimchi to a mouse model improves the mental symptoms of a mouse such as depression and anxiety disorder, and have completed the present disclosure.

The *Lactobacillus sakei* strain according to the present disclosure is specifically a *Lactobacillus sakei* strain derived from kimchi, more specifically a *Lactobacillus sakei* WIKIM31 strain derived from kimchi. Although a strain deposited in the Korea Culture Center of Microorganisms with accession number KFCC11654P (domestic deposit) and/or KCCM12654P (international deposit) was used as the *Lactobacillus sakei* WIKIM31 strain in the present disclosure, the means of acquisition is not limited thereto.

In the present disclosure, the *Lactobacillus sakei* WIKIM31 strain deposited with the above accession number is understood to include the strain deposited with the accession number and a *Lactobacillus sakei* strain equivalent to the WIKIM31 strain. It is understood to include, for example, a *Lactobacillus sakei* strain which has some difference (mutation, substitution, insertion, deletion, etc.) in the gene sequence and a *Lactobacillus sakei* strain having specifically 90% or more homology, more specifically 95% or more homology, most specifically 99% or more homology, to the gene sequence of the WIKIM31 strain.

The homology between *Lactobacillus sakei* strains may be analyzed by molecular biological identification and characterization techniques (Tenover et al., J. Clin. Microbiol., 1995, 33(9), 2233-2239, 1995), PCR and various electrophoresis techniques (Walter et al., Appl. Environ. Microbiol., 2001, 67(6), 2578-2585), RAPD (randomly amplification of polymorphic DNA) (Cusick et al., Appl. Environ. Microbiol., 2001, 66(5), 2227-2231), PCR using species-specific primers (species-specific PCR, SS-PCR) (Matsuki et al., Appl. Environ. Microbiol., 1999, 65(10), 4506-4512), etc. widely known in the art. 16S and 23S rRNAs and the interspacial region of 16S-23S rRNA have been studied a lot as the major target genes of SS-PCR and their base sequence data are published in various web-based databases (Naimi et al., Microbiol., 1997, 143(6), 823-834).

The *Lactobacillus sakei* WIKIM31 strain of the present disclosure is a Gram-positive, rod-shaped, facultative anaerobe which can grow under both aerobic and anaerobic conditions.

The present disclosure provides a composition containing *Lactobacillus sakei,* a culture thereof, a lysate thereof, a fermentation product thereof or an extract thereof.

According to a specific exemplary embodiment of the present disclosure, the *Lactobacillus sakei* strain of the present disclosure is *Lactobacillus sakei* WIKIM31.

The *Lactobacillus sakei* WIKIM31 contained in the composition according to the present disclosure may be in live or killed state and may also be in dried or freeze-dried form. The forms of lactic acid bacteria suitable for inclusion in various compositions and methods for preparing the same are well known to those skilled in the art. For example, the *Lactobacillus sakei* WIKIM31 may be prepared into a culture obtained by culturing using a known liquid or solid medium, a concentrate of the culture, a dried product of the culture, a fermentation product obtained by culturing together with additional ingredients, an extract obtained by extracting the strain with an organic solvent, or a lysate obtained by lysing or homogenizing the cell membrane of the strain, although not being limited thereto.

In the present disclosure, the composition may be a composition containing a live or killed *Lactobacillus sakei* WIKIM31 strain.

The present disclosure provides a food composition for preventing or alleviating depression or anxiety disorder, which contains *Lactobacillus sakei,* a culture thereof, a lysate thereof, a fermentation product thereof or an extract thereof as an active ingredient.

According to a specific exemplary embodiment of the present disclosure, the *Lactobacillus sakei* strain of the present disclosure is *Lactobacillus sakei* WIKIM31.

In the food composition, the *Lactobacillus sakei* WIKIM31 strain may be derived from kimchi, as described above.

In the present disclosure, "depression" refers to a neuropsychiatric disorder characterized mainly by sorrow, lack of taste, loss of feeling, anhedonia (loss of pleasure), illusion, delusion, etc. It refers to a disease which causes psychomotor retardation, pessimism, despair, suicidal ideation and suicidal attempt. The depression is accompanied by various physical symptoms such as loss of appetite, insomnia, constipation, decreased sexual desire, increased susceptibility to diseases due to declined immune function, etc. More specifically, it refers to the symptoms of decreased activity. And, "anxiety disorder" refer to a neuropsychiatric disorder characterized by physical or behavioral symptoms such as increased heart rate, shortness of breath, panic disorder, fear, sweating, etc. caused by the feelings of anxiety. More specifically, it refers to restlessness and the tendency to move toward a corner in an enclosed space, although not being limited thereto. Examples of the anxiety disorder include phobic disorder, panic disorder, generalized anxiety disorder, obsessive-compulsive disorder, etc.

In a specific exemplary embodiment, the depression or anxiety disorder may be depression or anxiety disorder occurring in a subject with a neurodegenerative disease.

The neurodegenerative disease may be one or more disease selected from a group consisting of Parkinson's disease, Alzheimer's disease, Huntington's disease, Lou Gehrig's disease (amyotrophic lateral sclerosis), Creutzfeldt-Jakob disease, stroke, multiple sclerosis, learning disorder, cognitive impairment and memory impairment, although not being limited thereto.

According to a specific exemplary embodiment of the present disclosure, the fermentation product may be a kimchi fermentation product or a fermentation product which is not a soy fermentation product.

In the present disclosure, in order to investigate the effect of the administration of *Lactobacillus sakei* WIKIM31 on the prevention or alleviation of depression or anxiety disorder, a *Lactobacillus sakei* WIKIM31 strain was administered after inducing depression in a normal animal model or a *Lactobacillus sakei* WIKIM31 strain was administered after inducing Parkinson's disease in an animal model, and then an open field test was conducted. As a result, overall movement, exploratory behavior and residence time in the center zone were increased in the test group to which the *Lactobacillus sakei* WIKIM31 strain of the present disclosure was administered as compared to a control group. Considering that individuals with depression or anxiety disorder show increased standstill time, it can be seen that the administration of the *Lactobacillus sakei* WIKIM31 strain can prevent depression or anxiety disorder since it increases movement.

When the composition of the present disclosure is used as a food composition, the food composition may be in the form of a functional health food, a condiment, a beverage, a bar, etc. In addition, the food composition containing the strain as an active ingredient may be a beverage such as fermented milk, etc. Therefore, the present disclosure provides a fermentation starter for acid bacteria, which contains *Lactobacillus sakei* WIKIM31 or a culture thereof.

The food composition of the present disclosure may be prepared using a sitologically suitable and physiologically acceptable adjuvant in addition to the active ingredient. The adjuvant may include an excipient, a disintegrant, a sweetener, a binder, a coating agent, a swelling agent, a lubricant, a glidant, a flavorant, etc.

The food composition may be formulated into a pharmaceutical composition for administration by adding one or more sitologically acceptable carrier in addition to the active ingredient described above.

For example, for formulation into a tablet or a capsule, the active ingredient may be used in combination with an oral, nontoxic, pharmaceutically acceptable inert carrier such as ethanol, glycerol, water, etc. And, if desired or necessary, a suitable binder, lubricant, disintegrant or coloring agent may also be included. The suitable binder includes a natural sugar such as starch, gelatin, glucose or β-lactose, a corn sweetener, a natural or synthetic gum such as acacia, tragacanth or sodium oleate, sodium stearate, magnesium stearate, sodium benzoate, sodium acetate, sodium chloride, etc., although not being limited thereto. The disintegrant includes starch, methyl cellulose, agar, bentonite, xanthan gum, etc., although not being limited thereto. As an acceptable pharmaceutical carrier in a composition formulated into a liquid solution, one or more ingredient of saline, sterile water, Ringer's solution, buffered saline, albumin injection solution, dextrose solution, maltodextrin solution, glycerol and ethanol, which are sterilized and suitable for the living body, may be mixed. Other conventional additives such as an antioxidant, a buffer, a bacteriostat, etc. may be added, if necessary. In addition, a diluent, a dispersant, a surfactant, a binder or a lubricant may be added additionally for formulation into an injectable formulation such as an aqueous solution, a suspension, an emulsion, etc., a pill, a capsule, a granule or a tablet.

The food composition according to the present disclosure may be added to various foods. The foods to which the composition of the present disclosure can be added include, for example, beverages, vitamin complexes, dietary supplements, etc.

The food composition of the present disclosure may contain ingredients commonly added when preparing foods, for example, a protein, a carbohydrate, a fat, a nutrient, a flavoring agent and a flavorant. Examples of the carbohydrate include common sugars such as a monosaccharide, e.g., glucose, fructose, etc., a disaccharide, e.g., maltose, sucrose, oligosaccharides, etc., a polysaccharide, e.g., dextrin, cyclodextrin, etc. and sugar alcohols such as xylitol, sorbitol, erythritol, etc. As the flavorant, a natural flavorant (thaumatin, stevia extract [e.g., rebaudioside A, glycyrrhizin, etc.]) or a synthetic flavorant (saccharin, aspartame, etc.) may be used. For example, when the food composition of the present disclosure is prepared into a drink or a beverage, citric acid, fructose syrup, sugar, glucose, acetic acid, malic acid, fruit juice, plant extracts, etc. may be further contained.

In addition, the present disclosure provides a pharmaceutical composition for preventing or treating depression or anxiety disorder, which contains *Lactobacillus sakei,* a culture thereof, a lysate thereof, a fermentation product thereof or an extract thereof as an active ingredient.

According to a specific exemplary embodiment of the present disclosure, the *Lactobacillus sakei* strain of the present disclosure is *Lactobacillus sakei* WIKIM31.

The present disclosure also provides a method for treating depression or anxiety disorder, which includes administering a therapeutically effective amount of *Lactobacillus sakei,* a culture thereof, a lysate thereof, a fermentation product thereof or an extract thereof to a subject in need thereof.

According to a specific exemplary embodiment of the present disclosure, the fermentation product may be a kimchi fermentation product or a fermentation product which is not a soy fermentation product.

The term "subject" used herein refers to a mammal which is the subject of treatment, observation or experiment, specifically human or an animal in need of prevention and/or treatment of depression or anxiety disorder.

In a specific exemplary embodiment, the subject may be a subject having a neurodegenerative disease which exhibits the symptoms of depression or anxiety disorder.

In addition, the term "neurodegenerative disease" used herein may refer to one or more disease selected from a group consisting of Parkinson's disease, Alzheimer's disease, Huntington's disease, Lou Gehrig's disease (amyotrophic lateral sclerosis), Creutzfeldt-Jakob disease, stroke, multiple sclerosis, learning disorder, cognitive impairment and memory impairment, although not being limited thereto.

In a specific exemplary embodiment, the neurodegenerative disease may be Parkinson's disease.

In an example described below, as a result of inducing Parkinson's disease in a group to which a *Lactobacillus sakei* WIKIM31 strain was induced, it was confirmed that overall movement, exploratory behavior and residence time in the center zone are increased as compared to a negative control group (PBS-ingested group). That is to say, it was confirmed that the present disclosure can be used for preventing, alleviating or treating depression or anxiety disorder since the *Lactobacillus sakei* WIKIM31 strain alleviates depression or anxiety disorder in a mouse mode of a neurodegenerative disease.

The pharmaceutical composition according to the present disclosure may be administered by common methods via oral, intravenous, intraarterial, intraperitoneal, intramuscular or intrasternal routes.

The pharmaceutical composition of the present disclosure may contain a pharmaceutically acceptable carrier. In the present disclosure, the term "pharmaceutically acceptable carrier" refers to a carrier or a diluent which does not cause an intolerable side effect and allows the active ingredient to retain its biological activity and characteristics. In the present disclosure, the pharmaceutically acceptable carrier may be one or more of saline, sterile water, Ringer's solution, buffered saline, dextrose solution, maltodextrin solution, glycerol and ethanol. If necessary, another common additive such as an antioxidant, a buffer, a bacteriostat, etc. may be added to formulate an injection suitable for injection to a tissue or organ. In addition, it may be formulated into a dried preparation (particularly, a freeze-dried preparation) which can be prepared into an injectable solution by adding an isotonic sterile solution or, if necessary, sterile water or physiological saline. In addition, a target organ-specific antibody or ligand may be bound to the carrier for specific action on a target organ. Suitable preparations known in the art are described in Remington's Pharmaceutical Science (Mack Publishing Company, Easton PA).

In addition, the composition of the present disclosure may specifically further contain a filler, an excipient, a disintegrant, a binder, a glidant, etc. In addition, the composition of the present disclosure may be formulated using a method known in the art such that the active ingredient is released immediately, in a sustained manner or in a delayed manner after being administered into a mammal.

In the present disclosure, "administration" refers to introduction of the composition of the present disclosure to a patient by any suitable method. The composition of the present disclosure may be administered via various oral or parenteral routes that can access the target tissue. It may be administered intraperitoneally, intravenously, intramuscularly, subcutaneously, intradermally, orally, topically, intranasally, intrapulmonarily or intrarectally, although not being limited thereto.

For example, the composition of the present disclosure may be administered by intramuscular, intravenous or intraperitoneal injection for clinical administration.

For injection, the composition may be specifically prepared into a pharmaceutically acceptable buffer such as Hank's solution, Ringer's solution or physiological saline buffer. For transmucosal administration, a non-penetrating agent suitable for penetration into a barrier is used. The non-penetrating agent is generally known in the art.

Preparations for parenteral administration include a sterilized aqueous solution, a nonaqueous solution, a suspension, an emulsion, etc. For the nonaqueous solution or suspension, propylene glycol, polyethylene glycol, vegetable oil such as olive oil, injectable ester such as ethyl oleate, etc. may be used.

In the present disclosure, "effective amount" refers to an amount necessary to delay or completely stop the onset or progression of a particular disease to be treated, and the effective amount of the *Lactobacillus sakei* WIKIM31 contained in the pharmaceutical composition of the present disclosure means the amount required to achieve the effect of preventing or treating depression or anxiety disorder. Accordingly, the effective amount may be adjusted depending on various factors including the type of a disease, the severity of the disease, the types and contents of other ingredients contained in the composition, the age, body weight, general health condition, sex and diet of a patient, administration time, administration route, treatment period and co-administered drugs. It is obvious to those skilled in the art that a suitable daily dosage can be determined by a prescriber within the proper medical determination range.

For the objectives of the present disclosure, it is preferred that a specific therapeutically effective amount for a particular patient varies depending on various factors including the type and degree of a reaction to be achieved, the specific composition in which other ingredients may be used in some cases, the age, body weight, general health condition, sex and diet of a patient, administration time, administration route, the excretion rate of the composition, treatment period, co-administered drugs and similar factors well known in the art.

In the present disclosure, "treatment" refers to an approach to obtain beneficial or desirable clinical outcomes. For the purposes of the present disclosure, the beneficial or desirable clinical outcomes include, but are not limited to, alleviation of symptoms, diminishment of the extent of a disease, stabilization of the disease state (i.e., prevention of worsening), delay or slowing of disease progression, amelioration or temporary palliation of the disease state, and remission (whether partial or total), whether detectable or undetectable. In addition, the "treatment" may mean prolonging survival rate as compared to the expected survival rate when the treatment is not given. The "treatment" refers to both therapeutic treatment and prophylactic treatment. The treatment includes not only the prevention of a disorder but also the treatment required for a disorder that has occurred already. The "palliation" of a disease means diminishing the extent of a disease state and/or undesirable clinical symptoms and/or slowing or extending the time course of disease progression as compared to when the treatment is not given.

The food or pharmaceutical composition according to the present disclosure may contain the *Lactobacillus sakei* strain in an amount of about 10²-10¹⁵ CFU/mL, specifically 10⁶-10¹² CFU/mL, more specifically 10⁷-10¹¹ CFU/mL, most specifically 10⁸-10¹⁰ CFU/mL, based on a single dose. The strain may be administered in live or killed state, and may be killed or attenuated prior to ingestion. In addition, it may further pass through a pasteurization process of heating. The amount of the strain necessary for providing the minimum effect and the recommended daily intake may be generally about 10²-10¹⁵ CFU/mL, although it can vary depending on the physical or health condition of the recipient.

Depression or anxiety disorder may be prevented or alleviated when the food or pharmaceutical composition according to the present disclosure is administered to a subject 3 or more times a week, specifically 5 or more times a week.

The advantages and features of the present disclosure and methods for achieving them will become more apparent by the examples descried below. However, the present disclosure is not limited by the examples descried below but may be embodied in various other forms. The examples are merely provided such that the disclosure of the present disclosure is complete and those having ordinary knowledge in the art to which the present disclosure belongs can fully understand the scope of the present disclosure. The present disclosure is defined by the appended claims only.

### [Advantageous Effects]

A *Lactobacillus sakei* strain according to the present disclosure can be used for preventing, alleviating or treating various mental disorders including depression and anxiety disorder.

### [Brief Description of Drawings]

FIG. 1 shows a result of conducting open field test on a mouse model of Parkinson's disease (brown: general movement of mouse (movement in X and Y axes detected by far infrared sensor); blue: exploratory behavior of mouse (movement in X, Y and Z₁ axes detected by far infrared sensor), Naive: non-treatment group, MPTP: negative control group (Parkinson's disease induced after ingestion of PBS), WIKIM31: WIKIM31 ingestion group (Parkinson's disease induced after ingestion of WIKIM31)).
FIG. 2 shows a result of conducting open field test on a mouse model of Parkinson's disease. (a) shows the total distance traveled by a mouse in a plastic cage, (b) shows residence time in the center zone, and (c) shows rearing time during which the mouse stood on hind legs.
FIG. 3 shows a result of analyzing the blood level of corticosterone which is a stress hormone in a rat model of general depression and/or anxiety disorder.
FIG. 4 shows a result of conducting open field test on a rat model of general depression and/or anxiety disorder.
FIG. 5 shows a result of conducting forced swim test on a rat model of general depression and/or anxiety disorder.

### [Best Mode]

Hereinafter, the present disclosure is described in detail through examples. The following examples merely illustrate the present disclosure, and the scope of the present disclosure is not limited by the examples.

### [Examples]

### Example 1: Preparation of culture of Lactobacillus sakei WIKIM31 strain

A *Lactobacillus sakei* WIKIM31 strain, which is derived from kimchi and is deposited in the Korea Culture Center of Microorganisms with the accession numbers KFCC11654P (domestic deposit) and KCCM12654P (international deposit), was used for experiment. After culturing the *Lactobacillus sakei* WIKIM31 strain in MRS medium at 30 °C for 24 hours, the cells were centrifuged at 8,000 rpm for 5 minutes and the remaining medium was removed by rinsing with PBS. 1×10¹⁰ CFU/mL of cells were quantitated using PBS. 0.2 mL (1×10⁹ CFU) was orally administered to an experimental animal 5 times a week. Sterilized PBS was administered to negative and positive control groups.

### Example 2: Investigation of effect of preventing or alleviating depression and anxiety disorder in animal model

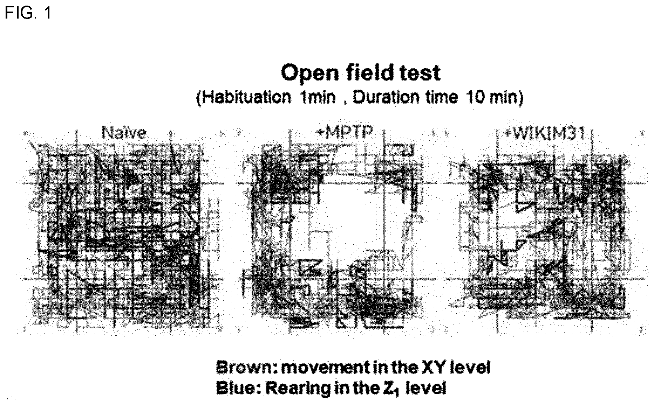

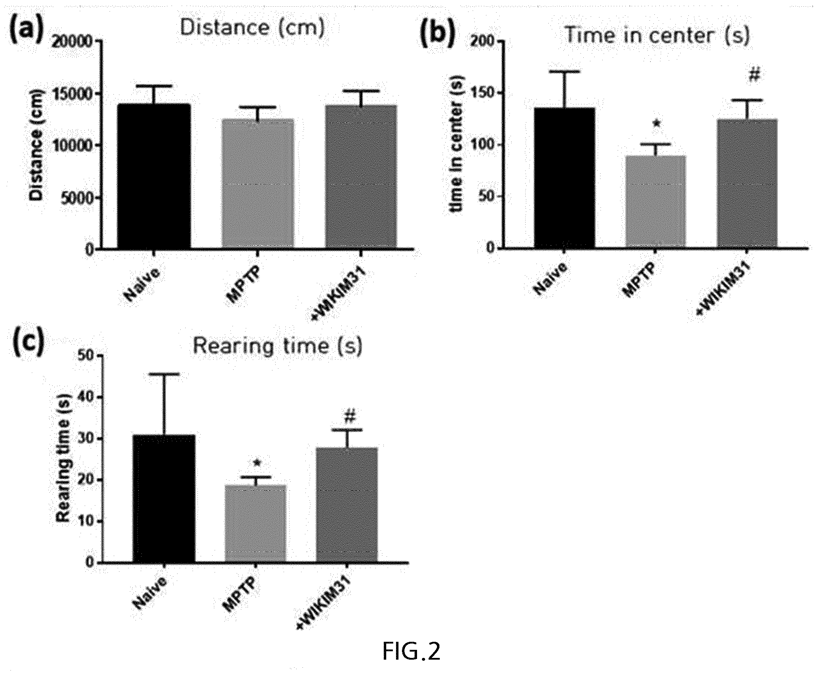

After accustoming 8-week-old male mice (C57BL/6) in a laboratory room for a week, *Lactobacillus sakei* WIKIM31 (KFCC11654P) was orally administered for 30 days. Then, after inducing Parkinson's disease by intraperitoneally injecting MPTP (1-methyl-4-phenyl-1,2,3,6-tetrahydropyridine), the effect of *Lactobacillus sakei* WIKIM31 was investigated. The result is shown in FIGS. 1 and 2. In FIGS. 1 and 2, Naive stands for a normal mouse model, MPTP a mouse model with Parkinson's disease induced after oral administration of PBS, and WIKIM31 a mouse model with Parkinson's disease induced after oral administration of *Lactobacillus sakei* WIKIM31.

Open field test was conducted to evaluate the symptoms of anxiety in an animal model of Parkinson's disease. The open field test was conducted by exposing an experimental animal to a new spacious environment with no place to escape or hide and the level of anxiety of the experimental animal was investigated by monitoring exploratory behavior. A plastic cage (45 cm × 45 cm × 40 cm) used for the open field test was divided into 3 × 3 zones. The anxiety level of the experimental animal was estimated by analyzing the zones where the mouse resided within the open field, the activity of the mouse such as traveled distance, and the rearing time during which the mouse stood on hind legs for exploratory behavior. The behavior of the animal was monitored using the TSE multi-conditioning system (TSE Systems, USA) and the Actimot video tracking system. A video camera was installed in the center of the ceiling at a height of 100 cm from the bottom so as to record each plastic cage (45 cm × 45 cm × 40 cm) for the open field test. The location of the animal in the open field was analyzed automatically using a far infrared sensor of the ActiMot frame (TSE Systems, USA). After accustoming the mouse in the plastic cage for 1 minute, its behavior was recorded for 10 minutes. The center zone of the plastic cage was set to 15 cm × 15 cm.

As a result of the open field test, it was confirmed that overall movement was increased (FIG. 1 and FIG. 2 (a)) and residence time in the center zone was increased (FIG. 2 (b)) in the *Lactobacillus sakei* WIKIM31 ingestion group as compared to the PBS-treated negative control group. In addition, the time spent on exploratory behavior was increased significantly as compared to the negative control group (FIG. 2 (c)). Therefore, it was confirmed that the ingestion of *Lactobacillus sakei* WIKIM31 significantly alleviates the symptoms of depression and anxiety disorder induced by MPTP (p < 0.05).

### Example 3: Investigation of effect of preventing or alleviating symptoms of depression and anxiety disorder in normal animal model

### 3-1. Induction of depression in normal animal model

After accustoming 6-week-old male rats (Sprague-Dawley) in a laboratory room for a week, chronic stress was applied from week 7. Stress was applied randomly to the experimental animals in a non-regular manner by depriving water or feed, providing empty water bottles, reversing dark-light cycles, flickering lights, providing noise frequently, providing wet bedding, tilting the cage, providing restrain stress, etc. every day. The stress was applied for a total of 6 weeks and different stress was applied every day. In the first two weeks, depression was induced by applying stress without ingestion of *Lactobacillus sakei* WIKIM31. After ingesting *Lactobacillus sakei* WIKIM31 (KCCM12654P) for the subsequent 4 weeks while applying stress, the alleviation of depression was measured.

### 3-2. Analysis of blood corticosterone level

After the induction of stress, blood corticosterone level was measured to evaluate of the antidepressant effect of the administered strain. The blood corticosterone level was analyzed using an ELISA kit (corticosterone assay, Ca:KGE009, RND Systems, USA) by extracting blood. The blood was taken via the jugular vein. 300 µL of whole blood was taken and serum was separated for analysis by centrifuging at 4,500 rpm for 10 minutes.

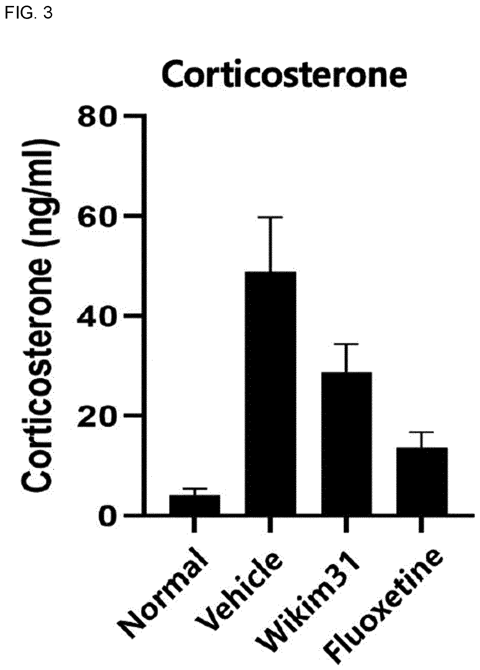

As a result of the blood corticosterone level analysis, it was confirmed that the stress hormone was decreased in the *Lactobacillus sakei* WIKIM31 ingestion group as compared to the PBS-treated negative control group (FIG. 3). Therefore, it was confirmed that the symptoms of depression and anxiety disorder induced by repeated stress can be alleviated through the ingestion of *Lactobacillus sakei* WIKIM31.

### 3-3. Open field test

Open field test was conducted to evaluate the symptoms of depression and anxiety in a depression-induced rat model. The open field test was conducted by exposing an experimental animal to a new spacious environment with no place to escape or hide and the level of depression of the experimental animal was investigated by monitoring behavior. The measurement was made in a cage of 50 cm × 50 cm × 50 cm for 15 minutes per rat. The center zone of the plastic cage was set to 25 cm × 25 cm and the distance traveled in the center zone was analyzed. A smaller ratio of the distance traveled in the center zone to the total distance traveled can be determined as depression. The behavior of the animal was monitored using a video tracking system (Smart 3.0, Panlab, USA).

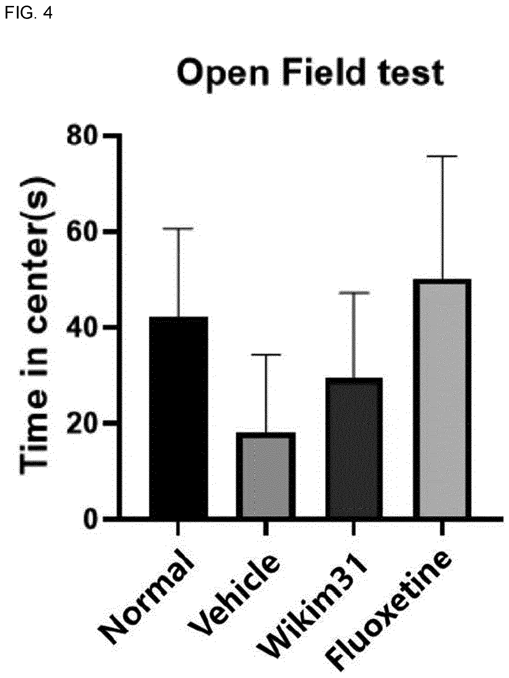

As a result of the open field test, it was confirmed that the residence time in the center zone was increased in the *Lactobacillus sakei* WIKIM31 ingestion group as compared to the PBS-treated negative control group (FIG. 4). Therefore, it was confirmed that the ingestion of *Lactobacillus sakei* WIKIM31 alleviates the symptoms of depression and anxiety disorder induced by repeated stress.

### 3-4. Forced swim test

Forced swim test was conducted for 10 minutes after filling water in a transparent acrylic cylinder (12 cm in diameter, 40 cm in height) to a height of 30 cm. After the experiment was completed, the animal was placed under an infrared lamp for 30 minutes for drying and then returned to the cage. The behavior of the animal during the 10 minutes was measured as immobility time and analyzed using an image analyzer (Smart 3.0, Panlab, USA).

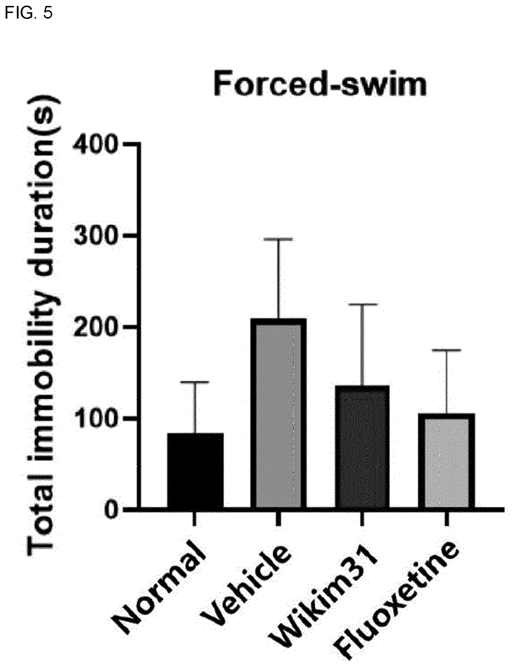

As a result of the forced swim test, it was confirmed that the time of forced swimming was decreased in the *Lactobacillus sakei* WIKIM31 ingestion group as compared to the PBS-treated negative control group (FIG. 5). Therefore, it was confirmed that the ingestion of *Lactobacillus sakei* WIKIM31 alleviates the symptoms of depression and anxiety disorder induced by repeated stress.

### [Accession numbers]

Depository agency: Korea Culture Center of Microorganisms (domestic)
Accession number: KFCC11654P
Date of deposition: 20160304
Depository agency: Korea Culture Center of Microorganisms (international)
Accession number: KCCM12654P
Date of deposition: 20200114

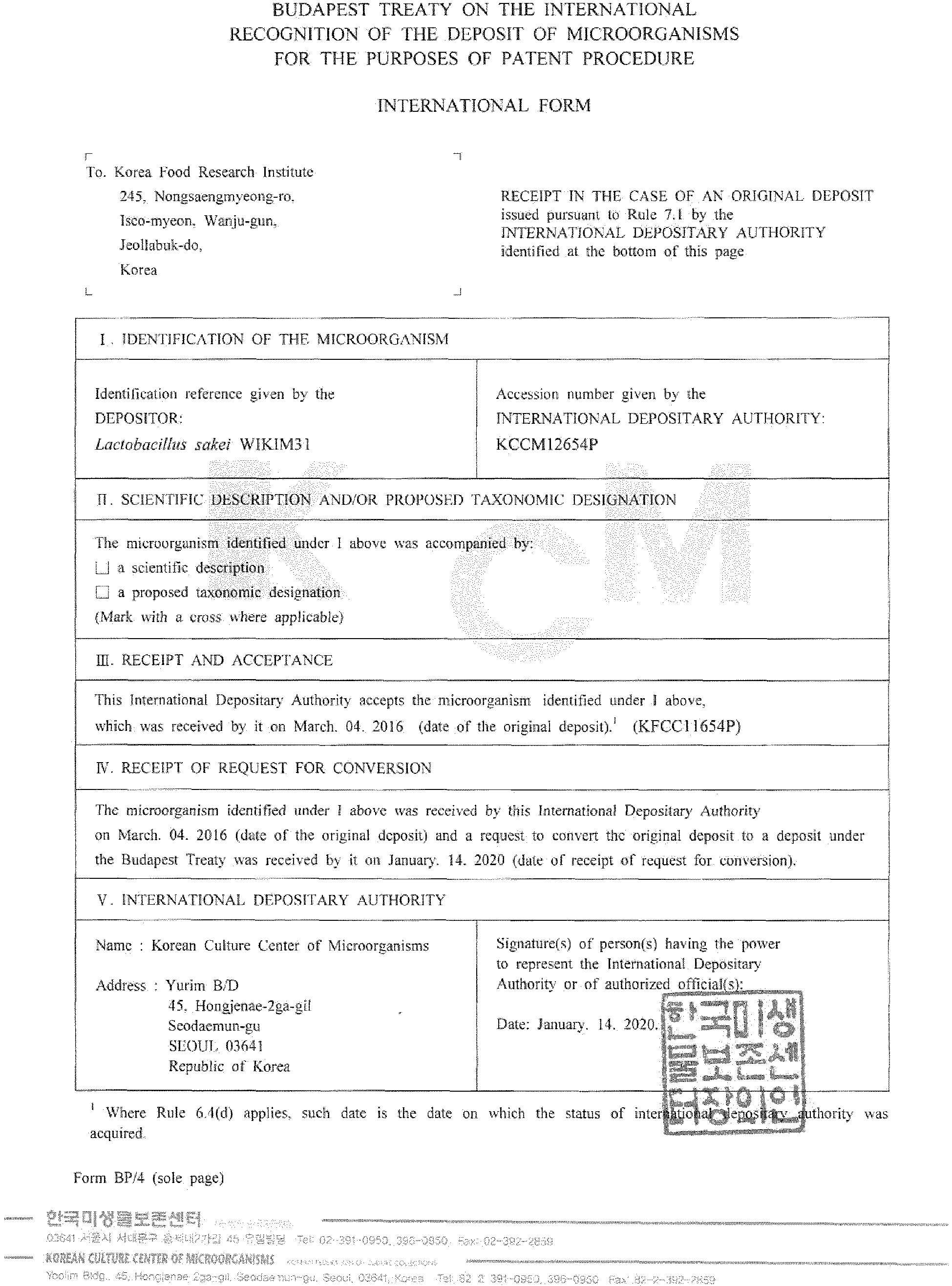

## Claims

1. A pharmaceutical composition for preventing or treating depression or anxiety disorder, comprising *Lactobacillus sakei,* a culture thereof, a lysate thereof, a fermentation product thereof or an extract thereof as an active ingredient.

2. The pharmaceutical composition according to claim 1, wherein the pharmaceutical composition comprises 1×10² to 1×10¹⁵ CFU/mL of *Lactobacillus sakei* and the pharmaceutical composition prevents or alleviates depression or anxiety disorder when administered to a subject 3 or more times a week.

3. The pharmaceutical composition according to claim 1, wherein the *Lactobacillus sakei* is *Lactobacillus sakei* WIKIM31 deposited with an accession number KCCM12654P.

4. The pharmaceutical composition according to claim 1, wherein the depression or anxiety disorder is depression or anxiety disorder in a subject having a neurodegenerative disease.

5. The pharmaceutical composition according to claim 4, wherein the neurodegenerative disease is one or more selected from a group consisting of Parkinson's disease, Alzheimer's disease, Huntington's disease, Lou Gehrig's disease (amyotrophic lateral sclerosis), Creutzfeldt-Jakob disease, stroke, multiple sclerosis, learning disorder, cognitive impairment and memory impairment.

6. The pharmaceutical composition according to claim 1, wherein the pharmaceutical composition is administered via an administration route selected from oral administration, intravenous administration, intraperitoneal administration, intramuscular administration, subcutaneous administration, intradermal administration, topical administration, intranasal administration, intrapulmonary administration and intrarectal administration.

7. A food composition for preventing or alleviating depression or anxiety disorder, comprising *Lactobacillus sakei,* a culture thereof, a lysate thereof, a fermentation product thereof or an extract thereof as an active ingredient.

8. The food composition according to claim 7, wherein the *Lactobacillus sakei* is *Lactobacillus sakei* WIKIM31 deposited with an accession number KCCM12654P.

9. The food composition according to claim 7, wherein the food is a functional health food.

10. The food composition according to claim 7, wherein the food is a beverage, a bar or a fermented milk.

11. The food composition according to claim 7, wherein the depression or anxiety disorder is depression or anxiety disorder of a subject having a neurodegenerative disease.

12. The food composition according to claim 11, wherein the neurodegenerative disease is one or more selected from a group consisting of Parkinson's disease, Alzheimer's disease, Huntington's disease, Lou Gehrig's disease (amyotrophic lateral sclerosis), Creutzfeldt-Jakob disease, stroke, multiple sclerosis, learning disorder, cognitive impairment and memory impairment.

13. The food composition according to claim 7, wherein the food composition comprises 1×10² to 1×10¹⁵ CFU/mL of *Lactobacillus sakei* and the food composition prevents or alleviates depression or anxiety disorder when orally administered to a subject 3 or more times a week.
